Europäisches Patentamt

(19) European Patent Office

Office européen des brevets

(11) Publication number: **0 038 203**
**B1**

(12) **EUROPEAN PATENT SPECIFICATION**

(45) Date of publication of patent specification: 24.07.85

(51) Int. Cl.⁴: **A 61 M 1/34**

(21) Application number: **81301613.6**

(22) Date of filing: **13.04.81**

(54) Blood treatment apparatus.

(30) Priority: **16.04.80 JP 50735/80**
**16.04.80 JP 50736/80**

(43) Date of publication of application:
**21.10.81 Bulletin 81/42**

(45) Publication of the grant of the patent:
**24.07.85 Bulletin 85/30**

(84) Designated Contracting States:
**DE FR GB IT**

(56) References cited:
**DE-A-2 213 158**
**DE-A-2 642 535**
**DE-A-2 828 512**
**DE-A-2 845 399**
**DE-B-2 342 072**
**DE-U-7 013 380**

**JAPANESE JOURNAL OF MEDICAL**
**INSTRUMENTATION, Vol. 49, Supplement pp.**
**259-261 (1979)**

(73) Proprietor: **KURARAY CO., LTD.**
**1621 Sakazu**
**Kurashiki-City Okayama Prefecture (JP)**

(73) Proprietor: **KAWASUMI LABORATORIES INC.**
**2-7-15, Minamirokugo Otaku**
**Tokyo (JP)**

(72) Inventor: **Kawai, Syuji**
**1652-1, Sakazu**
**Kurashiki-city (JP)**
Inventor: **Yamane, Tadayuki**
**37, Koyamahoriike-cho Kita-ku**
**Kyoto-city (JP)**
Inventor: **Abe, Michio**
**3-3, Tokiwaminami-cho**
**Saiki-city Oita-pref. (JP)**
Inventor: **Ono, Toshihiko**
**2339, Oaza-osakamoto Yayoi-cho**
**Minamiamabe-gun Oita-pref. (JP)**
Inventor: **Yamauchi, Shuji**
**6171, Kitachisakurada**
**Saiki-city Oita-pref. (JP)**

(74) Representative: **Crampton, Keith John Allen**
**et al**
**D YOUNG & CO 10 Staple Inn**
**London WC1V 7RD (GB)**

### Description

This invention relates to blood treatment apparatus with an extracorporeal circuit. Various blood treatment techniques, *inter alia*, haemodialysis using a dialysis membrane, haemofiltration with a filtration membrane and a haemoperfusion with an adsorbent, have come into wide clinical use. Recently, a technique called plasmapheresis, which is an extracorporeal blood treatment technique, has been developed.

Plasmapheresis comprises first separating blood into plasma and corpuscular components and then treating the plasma to remove pathogenic factors. In more detail, plasmapheresis includes the plasma-exchange method, in which the plasma is exchanged for a plasma preparation, and the specific plasma component permeation method, which comprises further fractionating the plasma, removing the fraction giving rise to the problem, and returning the remaining fractions, together with the corpuscular components, to the circulation. When viewed as a therapeutic means, the plasma exchange method is not always preferable because the whole amount of the plasma should be exchanged and therefore a large amount of a plasma preparation and therefore great expense are required and because some adverse effects may be produced due to incomplete supplementation of various physiologic substances contained in the plasma. On the contrary, the specific plasma component permeation method can be regarded as a more desirable therapeutic means, since only a part of the plasma is discarded while the rest is returned to the circulation, whereby the two problems mentioned above can be greatly improved.

Pioneer studies of the specific plasma component permeation method have already been made and described, e.g. the report by T. Agishi et al. published in th Japanese Journal of Medical Instrumentation, vol. 49, Supplement pp. 259—261 (1979), and Japanese patent application laid open under No. 2444/1980, which discloses a blood treatment apparatus in which a specific plasma component permeation method is realized in combination with a water-removing means.

However, these known techniques are no more than proposals of possible or theoretical apparatus. No apparatus useful in medical practice is disclosed.

The present invention has resulted from intensive research on the specific plasma component permeation method using a membrane.

The present invention provides a blood treatment apparatus with a first and a second membrane module, which comprises a blood inlet circuit including a first pump $M_1$, the first membrane module including a semipermeable membrane having pores of a size for retaining a blood corpuscular fraction and for permeation of a plasma fraction connected to the blood inlet circuit, a plasma outlet circuit for the plasma separated by the first membrane module and including a pressure adjustment unit comprising at least a second pump $M_2$ for adjusting the plasma pressure so that it is within the range of from −5320 to +5320 Pa −40 mmHg to 40 mmHg, the second membrane module including a semipermeable membrane that has pores of a size to allow permeation of human blood serum albumin for plasma fractionation and that fractionates the plasma from the plasma outlet circuit into two fractions, a plasma fraction outlet circuit for expelling a high-molecular-weight fraction separated in the second membrane module and including a third pump $M_3$ positioned downstream of the second membrane module, a blood return circuit for receiving a low-molecular-weight fraction containing human blood serum albumin separated in the second membrane module, which circuit is united with a blood corpuscles outlet circuit for the fraction separated in the first membrane module, in order to combine the two fractions, and means for controlling the ratio between the rate of flow of the plasma flowing into the second membrane module and the rate of the plasma fraction to be expelled from it to a predetermined value by controlling the flow rate ratio between the second and third pumps $M_2$ and $M_3$.

In the apparatus in accordance with the present invention, separation of blood into plasma and corpuscular components and separation of the plasma into a high-molecular-weight and a low-molecular-weight fraction is carried out using two membrane modules of different kinds. The membrane to be used in the first membrane module, i.e. to be used for separating corpuscular components from plasma, is preferably a microporous membrane having an average effective pore size of 0.02—0.4 μm, preferably about 0.1 μm. It is preferably a homogeneous microporous membrane, a microfiltration membrane or a so-called asymmetrical membrane comprising a porous supporting layer and a relatively dense microporous layer. Pore sizes larger than 0.4 μm often lead to a haemolysis, whereas pore sizes smaller than 0.02 μm cut off such proteins as γ-globulin, and hence cannot give a plasma containing these proteins. Examples of such membranes are substantially uniform microporous membranes made of polyvinyl alcohol (PVA) polymers, as well as well as other substantially uniform microporous membranes and asymmetrical membranes made of ethylene-vinyl alcohol (EVA) copolymers, cellulose derivatives (e.g. cellulose acetates), polyolefins, polyacrylonitriles, polyamides, polyesters and polysulphones. Preferred among these are PVA, EVA, cellulose derivative and polysulphone membranes, which have good biocompatibility.

The membrane used in the second membrane module separates plasma into a high-molecular-weight fraction and a low-molecular-weight fraction. The boundary molecular weight can optionally be set depending on the desired purpose, depending on the molecular weight of the pathogen to be removed. For example, the apparatus of the present invention can be used in the treatment of autoimmune diseases, in which pathogenic substances are often present bound to γ-globulin having a molecular weight of about 160,000. Therefore, it is desirable that substances having molecular weights of about 160,000 and higher be removed but substances having lower molecular weights such as albumin, which is useful to the organism and has a molecular weight of 67,000, be returned. Thus, setting the boundary molecular weight at 100,000 can result in rigid separation of the above-mentioned γ-globulin and albumin. In another case, where an immune complex is the causative factor, the boundary of molecular-weight cut-off is set at 100,000—200,000.

As the second membrane, there can be used any membrane that can fractionate plasma under pressure. In this sense, a large selection of membranes having ultrafiltration capacity can be used. No special limitations are placed on the membrane structure, and the above-mentioned uniform microporous membranes, asymmetrical membranes and uniform gel membranes can be used. The term "uniform gel membranes" as used herein means membranes having substantially no micropores or tiny gap structures among joined particles when observed in the dry or wet state under an electron microscope even at a magnification of 24,000.

The membranes mentioned above are used in the form of flat membranes or hollow-fibre membranes and constitute membrane modules. In view of ease of module preparation and possibility of miniaturization, hollow-fibre membranes are preferred.

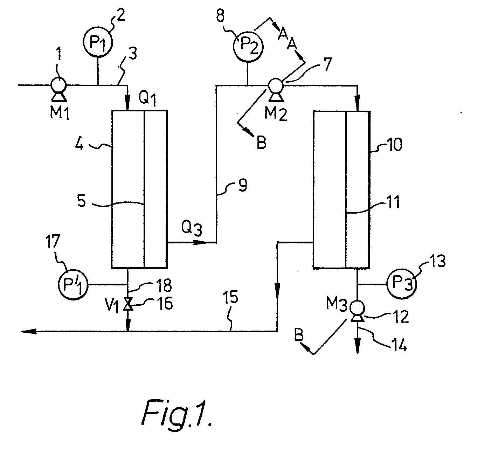

In the accompanying drawings, each of Figs. 1, 2 and 3 is a schematic representation of an example of blood treatment apparatus in accordance with the present invention.

Referring now to Fig. 1 of the drawings, blood is pumped through blood inlet circuit 3 equipped with pump $M_1$ (1) and preferably also pressure gauge $P_1$ (2) into plasma separation membrane module 4, where membrane 5 separates the blood into plasma and corpulsular components. The plasma separated is pumped through plasma outlet circuit 9 equipped with pressure gauge $P_2$ (8) and pump $M_2$ (7) into plasma fractionation membrane module 10. In the second membrane module, the plasma is fractionated by membrane 11, and the high-molecular-weight fraction is expelled from the system through plasma fraction outlet circuit 14 equipped with pump $M_3$ (12) and preferably also pressure gauge $P_3$ for monitoring (13). The low-molecular-weight fraction which has permeated membrane 11 flows out through blood return circuit 15. The corpuscular components coming from first membrane module 4 run through corpuscles outlet circuit 18 equipped with valve $V_1$ (16) and preferably also pressure gauge $P_1'$ for monitoring (17) and are combined with the low-molecular-weight plasma fraction coming through blood return circuit 15, to be returned to the organism.

Fig. 1 shows only those constituents that are essential in the practice of the present invention. Other equipment such as a drip chamber, a blood filter, a heparin infusion circuit, and another plasma treatment module, e.g. an activated carbon column, may also be included.

In using the apparatus shown in Fig. 1, the pressure indicated by pressure gauge $P_2$ (8) is adjusted to a predetermined constant value by correlating pressure gauge $P_2$ (8) with pump $M_2$ (7), so that it can never become substantially negative. For example, the number of revolutions of pump $M_2$ is adjusted by an electric signal corresponding to the pressure indication of pressure gauge $P_2$ so that the pressure on $P_2$ can be kept constant. A characteristic feature of the apparatus of the present invention is that the apparatus is constructed so as to maintain the pressure in the plasma outlet circuit at a constant level. Since the pressure exerted on the blood in the first membrane module is also kept constant, this construction is very desirable for the purpose of preventing blood corpuscles from being damaged. It is also desirable that the pressure ($P_1$) exerted on blood by first pump $M_1$ (1) be maintained at levels not exceeding a certain value, generally at 150 mmHg or below, preferably at 100 mmHg or below. When ease of handling in practice is taken into consideration, the lowest limit value for $P_1$ is about 0 mmHg. In addition to such conditions, it is also necessary to prevent the pressure on $P_2$ in the plasma outlet circuit from becoming substantially negative. As used in the present specification, a pressure that is not substantially negative is from −5320 to +5320 Pa −40 mmHg to 40 mmHg), preferably −2660 to +2660 Pa (−20 mmHg to 20 mmHg). It is desirable that the pressure be in the neighbourhood of 0 mmHg, i.e. atmosperic pressure. That the pressure on $P_2$ becomes more negative means that a negative pressure is exerted also on first membrane 5 and the trans-membrane pressure (TMP) increases. The increased TMP also increases the differential pressure exerted on the corpuscular components on the membrane surface, whereby the risk of corpuscles being damaged is increased. Therefore, such excessively negative pressure on $P_2$ should be avoided. The TMP should preferably be not greater than 100 mmHg. Furthermore, a secondary effect possibly produced by negative pressure in a blood treatment apparatus is that, if a part of the circuit should be connected loosely, a foreign substance such as air might penetrate into the circuit from the outside, which could lead to serious consequences. Adjustment of the pressure on $P_2$ so as not to become substantially negative is desirable also for prevention of such an accident.

Both first membrane 5 and second membrane 11 change in performance with time due to the adhesion of materials such as proteins during blood treatment. Therefore, when the blood pressure exerted on membrane 5 is kept constant, the amount of plasma separated ($Q_3$) changes with time. Since the second membrane module fractionates plasma under pressure, a change in the amount of plasma entering the second membrane module will cause a change in the pressure condition and consequently a change in the fractionation performance of the second membrane module. Such a change must be avoided at any cost. For this end, the flow rate ratio between pump $M_2$ (7) and pump $M_3$ (12) must be adjusted to a constant value so that the pressure in the second membrane module can be kept constant even if the amount of plasma $Q_3$ is changing. Furthermore, for safety reasons, it is desirable to provide the plasma fraction outlet circuit with pressure gauge $P_3$ (13) for directly monitoring the pressure in the second membrane module.

With a blood treatment apparatus constructed in the above manner, very good results can be obtained with the least possible influence on the blood, since it is possible to treat blood in a stable manner under conditions which produce a constant pressure on the blood, without any substantial change in the fractionation behaviour of the plasma.

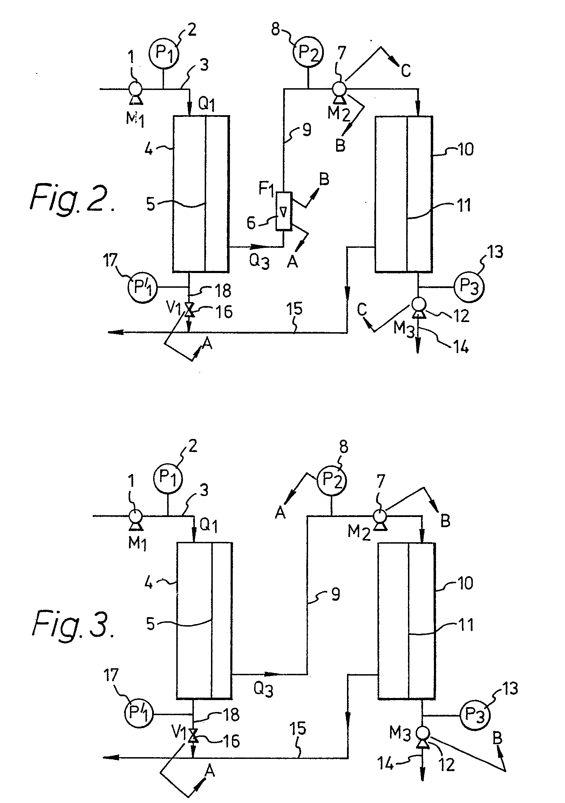

Referring now to Fig. 2, blood is pumped through blood inlet circuit 3 equipped with pump $M_1$ (1) and preferably also with pressure gauge $P_1$ (2) into plasma separation membrane module 4, where the blood is separated into plasma and a corpuscular fraction by membrane 5. The plasma separated is pumped through plasma outlet circuit 9, which is equipped with flowmeter $F_1$ (6), pump $M_2$ (7) and preferably also pressure gauge $P_2$ (8), into plasma fractionation membrane module 10. In the second membrane module, the plasma is fractionated by membrane 11, and the high-molecular-weight fraction is expelled through plasma fraction outlet circuit 14, which is equipped with pump $M_3$ (12) and preferably also with pressure gauge $P_3$ (13). The low-molecular-weight fraction that has permeated membrane 11 is led out of the second membrane module through blood return circuit 15. The corpuscular fraction coming from first membrane module 4 runs through corpuscles outlet circuit 18, which is equipped with valve $V_1$ (16) and preferably also with pressure gauge P'1 (17), and is combined with the low-molecular-weight plasma fraction coming through blood return circuit 15, and the mixture is returned to the circulatory system of the organism.

Fig. 2 shows only those constituents that are essential in the practice of the present invention. Other equipment such as a drip chamber, a blood filter, a heparin infusion circuit and another plasma treatment module, e.g. activated carbon, may also be included. In the apparatus shown in Fig. 2, the amount, or rate of flow, $Q_3$, of plasma coming from first membrane module 4 is adjusted to a predetermined value by controlling valve $V_1$ (16) and flowmeter $F_1$ (6) in association with each other. Thus, valve 16 is adjusted by an appropriate means so that flowmeter 6 may indicate a constant value. For example, opening and closing of valve 16 is controlled by an electric signal corresponding to the data furnished by the flowmeter. What is important with the first membrane module is that, in the plasma separation, damaging of blood corpuscles and consequential haemolysis can be prevented. To this end, the pressure $P_1$ (2) produced by first pump $M_1$ (1) is adjusted to 150 mmHg or below, preferably 100 mmHg or below. The lowest limit value is, as mentioned previously, about 0 mmHg. For observing the change of said pressure, it is preferable to provide pressure gauge $P_1$ (2). Both first membrane 5 and second membrane 11 change in performance with time due *inter alia* to adhesion of proteins during blood treatment. Therefore, even when the blood pressure on membrane 5 is kept constant, the amount of plasma separated, $Q_3$, changes with time. To solve this problem, it is necessary to provide flowmeter $F_1$ for directly monitoring the amount $Q_3$ of plasma in the plasma outlet circuit.

A characteristic feature of the apparatus of the present invention is that the apparatus is constructed so as to maintain the amount $Q_3$ of plasma at a constant level. This feature is common to other embodiments of the invention to be mentioned later. When blood is treated at a constant plasma flow rate ($Q_3$), the subsequent plasma fractionation can also be effected at a constant flow rate, so that blood treatment can be conducted in a regular and therefore very therapeutically efficient manner.

Furthermore, in the apparatus shown in Fig. 2, the pressure in plasma outlet circuit 9 ($P_2$) must be adjusted so as not to become substantially negative by controlling flowmeter $F_1$ (6) and pump $M_2$ (7) in association with one another. A negative pressure in the circuit 9 produces a negative pressure on the first membrane 5, whereby the transmembrane pressure (TMP) increases and consequently the differential pressure acting on the blood corpuscles on the membrane surface increases. The result is an increase in the risk of corpuscles being damaged, which should be avoided. As mentioned previously, the TMP should preferably be not greater than 100 mmHg. Furthermore, a secondary effect possibly produced by a negative pressure in a blood treatment apparatus like the one in accordance with the invention is that, if a loosely connected part should be present in the circuit, foreign matter such as air could penetrate into the circuit from the outside, which might lead to serious consequences. To prevent such an accident, adjustment must be made so as not to allow a substantially negative pressure as above defined, by controlling flowmeter $F_1$ (6) and pump $M_2$ (7) in association with each other. A pressure in the neighbourhood of 0 mmHg, i.e. atmospheric pressure, is desirable.

The plasma introduced into plasma fractionation membrane module 10 is fractionated by membrane 11. Thus, the low-molecular-weight components permeate the membrane, while the high-molecular-weight components are excluded and expelled from the system through pump $M_3$. If the amount of plasma introduced, $Q_3$, is constant, the amount of permeating components is determined by the amount of high-molecular-weight components that are expelled. Therefore, the amount of low-molecular-weight components to be sent to the blood return circuit can be adjusted through the feed rate ratio between pump $M_2$ and pump $M_3$. Accordingly, in accordance with the present invention, the ratio of the amount of plasma introduced into module 10 to the amount of plasma withdrawn from that module is adjusted by controlling the flow rate ratio between pump $M_2$ (7) and pump $M_3$ (12). It is preferable to provide pressure gauge $P_3$(13) in the plasma fraction outlet circuit for monitoring the pressure in that circuit.

The low-molecular-weight components that have permeated second membrane 11 run through blood return circuit 15 and are combined with the corpuscular fraction from corpuscles outlet circuit 18 and returned to the organism.

Turning to Fig. 3, it will be evident on comparison with Fig. 2 that both the apparatuses shown have common parts, with respect to which repeated description is avoided.

The plasma fraction separated in first membrane module 4 is sent out into plasma outlet circuit 9, which is equipped with pressure gauge $P_2$ (8) and pump $M_2$ (7). As compared with the apparatus shown in Fig. 2, the apparatus shown in Fig. 3 differs in that flowmeter $F_1$ is absent and pressure gauge, $P_2$ is essential. In this apparatus, too, pump $M_2$ (7) is adjusted so that the plasma outlet amount, $Q_3$, can be kept at a predetermined value. Furthermore, pressure gauge $P_2$ (8) and valve $V_1$ (16) are controlled in association with one another so that the pressure within plasma outlet circuit 9 cannot become substantially negative (as above defined). In addition, the plasma fractionation rate in the second membrane module is controlled by controlling the flow rate ratio between pump $M_2$ (7) and pump $M_3$ (12). The apparatus shown in Fig. 3 is compsed of a smaller number of constituent parts as compared with the apparatus shown in Fig. 2, and has a simplified control circuit, and therefore reduction in manufacturing cost and facility of operation may be expected.

As a pressure adjustment unit comprising at least a pump (M2) for adjusting the plasma pressure so as not to become substantially negative, the pressure adjustment units as mentioned in Fig. 1, 2 and 3 are preferred, but the following pressure adjustment units are also used in this invention;

(1) a pressure adjustment unit without controlling flowmeter F1 (6) and pump M2 (7) in association with each other in Fig. 2.

(2) a pressure adjustment unit controlling pressure gauge P2 (8) and pump M2 (7) in association with each other instead of controlling flowmeter F1 (6) and pump M2 (7) in association with each other in Fig. 2.

(3) a pressure adjustment unit controlling pressure gauge P2 (3) and pump M2 (7) in association with each other in Fig. 3.

Furthermore, for supplementing that portion of plasma that has been removed in second filter 10, an albumin solution or hydroxyethyl-starch (HES) or other substitute fluids can be added to the treated plasma for introduction into the body of a patient. In the practice of the present invention, in introducing such a substitute fluid, it is preferable that the rates of flow through the pump for substitute fluid introduction and pump $M_3$ (12) provided in the high-molecular-weight fraction outlet circuit 14 should be controlled in association with each other so that the amount of the high-molecular-weight fraction withdrawn is equal to the amount of the substitute fluid introduced. Neither too much nor too little substitute fluid may be introduced into the body.

In practising the present invention, the associated control of pump $M_2$ (7) and pump $M_3$ (12) or of pump $M_3$ (12) and the pump for substitute fluid introduction is preferably carried out electrically. Another possibility is that different inside diameters can be given to the tubes connected to the respective pumps and the tubes are squeezed by one and the same driving roller, whereby the flow rates can be adjusted according to the respective tube diameters.

The treatment system of the present invention can effectively be used in the treatment of the blood of patients with the following disorders: autoimmune haemolytic anaemia, idiopathic thrombocytopenic purpura chronic glomerulonephritis, Goodpasture syndrome, systemic lupus erythematosus, and progressive systemic sclerosis.

The following examples further illustrate the present invention.

Example 1

A first membrane module was constructed by incorporating into a cylindrical cartridge a polyvinyl alcohol hollow fibre membrane having a substantially uniform microporous structure with an average pore size of 0.04 µm, an inside diameter of 400 µm and membrane thickness of 200 µm, the membrane area being 0.15 m$^2$. A second membrane module was prepared by incorporating into a cylindrical cartridge an ethylene-vinyl alcohol copolymer (EVA) hollow-fibre membrane having an asymmetrical structure comprising a porous support layer and a microporous layer with an average micropore diameter of 110 angstroms (11 nm), an inside diameter of 330 µm and a membrane thickness of 45 µm as disclosed in Japanese Published Patent Specification No. 35,969/1980, the membrane area being 0.9 m$^2$. These membrane modules were built into the circuit shown in Fig. 1, and the blood of a patient with peripheral circulatory disturbance due to corticotropin-releasing factor (CRF) and arteriosclerosis was treated.

For plasma separation in the first membrane module, the blood flow rate through pump $M_1$ was adjusted to 120 ml/minute, and the pressure on $P_1$ was maintained at 90 mmHg by adjusting valve $V_1$. The rate of flow of the plasma separated was about 30 ml/minute at the early stage but dropped to 20 ml/minute with the lapse of time. However, pump $M_2$ and pressure gauge $P_2$ were controlled in association with each other while monitoring by means of pressure gauge $P_2$, so that gauge $P_2$ always indicated a pressure within the range of 0 to −20 mmHg. In addition, pump $M_3$ and pump $M_2$ were controlled in association with each other, so that the flow rate ratio between them was always 1:4. In this manner, the blood could be treated continuously for 2 hours. As a result, the percentage immunoglobulin removal from the blood of the patient was 23%.

Example 2

A first membrane module was constructed by incorporating a polyvinyl alcohol hollow fibre membrane having a substantially uniform microporous structure with an average pore size of 0.04 µm, an inside diameter of 400 µm and a membrane thickness of 200 µm into a cylindrical cartridge, the membrane area being 0.15 m$^2$. Separately, a second membrane module was constructed by incorporating into a cylindrical cartridge an EVA hollow-fibre membrane having an asymmetric structure comprising a porous support layer and a microporous layer with an average micropore diameter of 110 angstroms (11 nm), an inside diameter of 330 µm and a membrane thickness of 45 µm (cf. Japanese Published Patent Specification No. 35,969/1980), the membrane area being 0.9 m$^2$. These membrane modules were built into the circuit shown in Fig. 2, and the blood of a patient with peripheral circulatory disturbance due to CRF and arteriosclerosis was treated.

For plasma separation in the first membrane module, plasma outlet rate $Q_3$ was adjusted to 24 ml/minute on the average by controlling pump $M_2$ while monitoring flowmeter $F_1$, and in addition, pump $M_1$ and valve $V_1$ were respectively adjusted so that pressure gauge $P_1$ always indicated 100 mmHg. The blood flow rate, $Q_1$, was in the neighbourhood of 100 ml/minute. The pressure gauge $P_2$ always indicated a pressure within the range of 0 to −20 mmHg.

For fractionation of the plasma components coming from the first membrane module at a rate of 24 ml/minute into the second membrane module, the flow rate ratio between pump $M_2$ and pump $M_3$ was adjusted to 4:1. Thus, the rate of discharge flow through pump $M_3$ was about 6 ml/minute. The low-molecular-weight plasma fraction permeated the second membrane at a rate of 18 ml/minute, and was combined with the concentrated corpuscular fraction and returned to the patient. After continuous blood treatment in this manner for 3 hours, the percent immunoglobulin removal from the blood of the patient was 34%, and almost no changes were observed in electrolytes. After the outbreak of the disease, the patient had complained of a pain in the tiptoe, but after one blood treatment with this apparatus, the patient was free from such pain.

Example 3

Membrane modules (first and second) of the same type as used in Example 2 were built into the circuit shown in Fig. 3 for treating the blood of a patient with rheumatoid arthritis, an autoimmune disease. For plasma separation in the first membrane module, the blood flow rate ($Q_1$) was adjusted to 120 ml/minute by controlling pump $M_1$, and the feed rate through pump $M_2$ was adjusted to 22 ml/minute. Furthermore, a circuit was provided for control of valve $V_1$ in association with pressure gauge $P_2$ such that valve $V_1$ was operated so as to maintain the indication of gauge $P_2$ within the range of 0 to −20 mmHg. The indication of pressure gauge $P_1$ at that time was 80 mmHg. Haemolysis observed in this plasma separation procedure was 1 mg/dl as free haemoglobin. Thus was achieved good plasma separation with almost no substantial haemolysis.

The plasma sent out by pump $M_2$ was deprived of high-molecular-weight components in the second membrane module, then combined with the concentrated blood corpuscular fraction and returned to the patient. In this second membrane filtration, analysis revealed that the ratio between albumin and γ-globulin (A/G) in the fluid expelled from the system by pump $M_3$ was 0.4:1. When compared with the ratio A/G (0.6:1) for the plasma before filtration, this analytical result clearly indicates that a considerable amount of γ-globulin had been removed from the blood.

The flow rate ratio between pump $M_2$ and pump $M_3$ was adjusted to 4:1. Thus, the rate of discharge flow through pump $M_3$ was about 5.5 ml/minute. The low-molecular-weight plasma fraction permeated the second membrane at a rate of 16.5 ml/minute, and was combined with the concentrated corpuscular fraction and returned to the patient.

**Claims**

1. A blood treatment apparatus with a first and a second membrane module (4, 10), which comprises a blood inlet circuit including a first pump (1), the first membrane module (4) including a semipermeable membrane (5) having pores of a size for retaining a blood corpuscular fraction and for permeation of a plasma fraction connected to the blood inlet circuit, a plasma outlet circuit (9) for the plasma separated by the first membrane module and including a pressure adjustment unit comprising at least a second pump (7) for adjusting the plasma pressure so that it is within the range of from −5320 to +5320 Pa (−40 mmHg to 40 mmHg), the second membrane module (10) including a semipermeable membrane (11) that has pores of a size to allow permeation of human blood serum albumin for plasma fractionation and that fractionates the plasma from the plasma outlet circuit (9) into two fractions, plasma fraction outlet circuit (14) for expelling a high-molecular-weight fraction separated in the second membrane means and including a third pump (12) positioned downstream of the second membrane module (10), a blood return circuit (15) for receiving a low-molecular-weight fraction containing human blood serum albumin separated in the second membrane module (10), which circuit (15) is united with a blood corpuscles outlet circuit (18) for the fraction separated in the first membrane module (4), in order to combine the two fractions, and means for controlling the ratio between the rate of flow of the plasma flowing into the second membrane module (10) and the rate of the plasma fraction to be expelled from it to a predetermined value by controlling the flow rate ratio between the second and third pumps (7, 12).

2. Blood treatment apparatus as claimed in Claim 1, in which the pressure adjustment unit includes a pressure gauge (8), as well as the second pump (7), which are controlled in association with each other so as to keep the pressure in the plasma outlet circuit (9) in the said range.

3. Blood treatment apparatus as claimed in Claim 1, in which the plasma outlet circuit for the plasma separated by the first membrane module includes a flowmeter (6) as well as the second pump (7) and the blood corpuscles outlet circuit (18) includes a valve (16) that is controlled in association with the flowmeter (6) so as to adjust the rate of flow of the plasma ($Q_3$) from the first membrane module (4) to a predetermined rate, the pump (7) being controlled in association with the flowmeter (6) so as to keep the pressure in the plasma outlet circuit (9) in the said range.

4. A blood treatment apparatus as claimed in Claim 1, in which the plasma outlet circuit for the plasma separated in the first membrane module includes a pressure gauge (8) as well as the pump (7), the blood corpuscles outlet circuit (18) includes a valve (16) for returning the blood to the circulation of the patient, the pump (7) is controlled so as to adjust the rate of flow of plasma ($Q_3$) from the first membrane module (4) to a predetermined rate and the valve (16) is controlled in association with the pressure gauge (8) to keep the pressure in the plasma outlet circuit (9) in the said range.

5. Apparatus as claimed in any preceding Claim, in which the pressure in the plasma outlet circuit (9) is adjusted to be in the range −2660 to +2660 Pa (−20 mmHg to 20 mmHg).

**Revendications**

1. Dispositif pour le traitement du sang avec un premier et un second module à membrane (4, 10) qui comprend un circuit d'entrée du sang comprenant une première pompe (1), le premier module à membrane (4) comprenant une membrane semi-perméable (5) ayant des pores dont la taille est telle qu'ils retiennent une fraction corpusculaire du sang et permettent la perméation d'une fraction de plasma, raccordé au circuit d'entrée du sang, un circuit de sortie du plasma (9) pour le plasma séparé par le premier module à membrane et comprenant une unité d'ajustement de la pression comprenant au moins une second pompe (7) pour ajuster la pression du plasma afin qu'elle soit dans la gamme de −5 320 à +5 320 Pa (−40 mmHg à 40 mmHg), le second module à membrane (10) comprenant une membrane semi-perméable (11) ayant des pores dont la taille est telle qu'ils permettent la perméation de la sérum-albumine du sang humain pour le fractionnement du plasma et qui fractionne le plasma du circuit de sortie du plasma (9) en deux fractions, un circuit de sortie de la fraction de plasma (14) pour chasser une fraction de poids moléculaire élevé séparée dans le second dispositif à membrane et comprenant une troisième pompe (12) placée en aval du second module à membrane (10), un circuit de retour au sang (15) pour recevoir une fraction de bas poids moléculaire contenant la sérum-albumine du sang humain séparée dans le second module à membrane (10) lequel circuit (15) est réuni à un circuit de sortie des corpuscles du sang (18) pour la fraction séparée dans le premier mobule à membrane (4), de façon à combiner les deux fractions, et un dispositif de régulation du rapport entre le débit du plasma s'écoulant dans le second module à membrane (10) et le débit de la fraction de plasma à en chasser, à une valeur prédéterminée par ajustement du rapport des débits entre la second et la troisième pompe (7, 12).

2. Dispositif pour le traitement du sang comme revendiqué dans la revendication 1, dans lequel l'unité de régulation de la pression comprend un manomètre (8), ainsi que la seconde pompe (7) qui sont ajustés en association mutuelle de façon à maintenir la pression dans le circuit de sortie du plasma (9) dans ladite gamme.

3. Dispositif pour le traitement du sang comme revendiqué dans la revendication 1 dans lequel le circuit de sortie du plasma pour le plasma séparé par le premier module à membrane comprend un débitmètre (6) ainsi que la seconde pompe (7) et le circuit de sortie des corpuscles du sang (18) comprend une vanne (16) qui est réglée en association avec le débitmètre (6) de façon à ajuster le débit du plasma ($Q_3$) provenant du premier module à membrane (4) à une valeur prédéterminée, la pompe (7) étant réglée en association avec le débitmètre (6) de façon à maintenir la pression dans le circuit de sortie du plasma (9) dans ladite gamme.

4. Dispositif pour le traitement du sang comme revendiqué dans la revendication 1 dans lequel le circuit de sortie du plasma pour le plasma séparé dans le premier module à membrane comprend un manomètre (8) ainsi que la pompe (7), le circuit de sortie des corpuscules du sang (18) comprend une vanne (16) pour retourner le sang à la circulation du malade, la pompe (7) est réglée de façon à ajuster le débit du plasma (Q₃) provenant du premier module à membrane (4) à une valeur prédéterminée et la vanne (16) est réglée en association avec le manomètre (8) pour maintenir la pression dans le circuit de sortie du plasma (9) dans ladite gamme.

5. Dispositif comme revendiqué dans l'une quelconque des revendications précédentes dans lequel la pression du circuit de sortie du plasma (9) est ajustée pour être dans la gamme −2 660 à +2 660 Pa (−20 mmHg à 20 mmHg).

**Patentansprüche**

1. Blut-Behandlungsvorrichtung mit einem ersten und einem zweiten Membranmodul (4, 10), die aufweist:

einen Blut-Einlaßkreist mit einer ersten Pumpe (1),

den mit dem Blut-Einlaßkreis verbundenen ersten Membranmodul (4) mit einer halbdurchlässigen Membran (5), die Poren mit einer Größe für die Zurückhaltung eines korpuskularen Blut-Bestandteils und für den Durchtritt eines Plasma-Bestandteils hat,

einen Plasma-Auslaßkreis (9) für das durch den ersten Membranmodul separierte Plasma, der eine Druckeinstelleinheit umfaßt mit zumindest einer zweiten Pumpe (7) für die derartige Einstellung des Plasma-Drucks, daß dieser innerhalb des Bereiches von −5320 bis +5320 Pa (−40 mmHg bis +40 mmHg) liegt,

den zweiten Membranmodul (10) mit einer halbdurchlässigen Membran (11), die für die Plasma-Fraktionierung Poren mit einer den Durchtritt des menschlichen Blutserums Albumin zulassenden Größe hat, und die das Plasma von dem Plasma-Auslaßkreis (9) in zwei Bestandteile fraktioniert,

einen Plasma-Fraktions-Auslaßkreis (14) für die Herausführung eines in der zweiten Membraneinrichtung separierten Bestandteils mit einem hohen Molekulargewicht, der eine in der Strömungsrichtung hinter dem zweiten Membranmodul (10) liegende dritte Pumpe (12) umfaßt,

einen Blut-Rückführkreis (15) für die Aufnahme eines das menschliche Blutserum Albumin enthaltenden, im zweiten Membranmodul (10) separierten Bestandteils mit einem niedrigen Molekulargewicht, wobei der Kreis (15) mit einem Blutkorpuskel-Auslaßkreis (18) für den im ersten Membranmodul (4) separierten Bestandteil verbunden ist, um die beiden Bestandteile zu vereinigen, und

eine Einrichtung für die Einstellung des Verhältnisses zwischen der Flußrate des in den zweiten Membranmodul (10) fließenden Plasmas und der Rate des aus diesem herauszuführenden Plasma-Bestandteils auf einen vorgegebenen Wert durch Steuerung des Verhältnisses zwischen den Flußraten der zweiten und dritten Pumpe (7, 12).

2. Blut-Behandlungsvorrichtung nach Anspruch 1, in der die Druck-Einstelleinheit ein Druckmeßinstrument (8) sowie die zweite Pumpe (7) aufweist, die in Verbindung miteinander gesteuert sind, um den Druck in dem Plasma-Auslaßkreis (9) in den genannten Bereich zu halten.

3. Blut-Behandlungsvorrichtung nach Anspruch 1, in der der Plasma-Auslaßkreis für das von dem ersten Membranmodul separierte Plasma einen Durchflußmesser (6) sowie die zweite Pumpe (7) aufweist, und der Blutkorpuskel-Auslaßkreis (18) ein Ventil (16) aufweist, das in Verbindung mit dem Durchflußmesser (6) gesteuert ist, um die Flußrate des Plasmas (Q₃) von dem ersten Membranmodul (4) auf einen vorgegebene Rate einzustellen, wobei die Pumpe (7) in Verbindung mit dem Durchflußmesser (6) gesteuert ist, um den Druck in dem Plasma-Auslaßkreis (9) in dem genannten Bereich zu halten.

4. Blut-Behandlungsvorrichtung nach Anspruch 1, in der der Plasma-Auslaßkreis für das in dem ersten Membranmodul separierte Plasma ein Druckmeßinstrument (8) sowie die Pumpe (7) aufweist, der Blutkorpuskel-Auslaßkreis (18) ein Ventil (16) für die Rückführung des Blutes in den Kreislauf des Patienten aufweist, die Pumpe (7) gesteuert ist, um die Flußrate des Plasma (Q₃) von dem ersten Membranmodul (4) auf eine vorgegebene Rate einzustellen, und das Ventil (16) in Verbindung mit den Druckmeßinstrument (8) gesteuert ist, um den Druck in dem Plasma-Auslaßkreis (9) in dem genannten Bereich zu halten.

5. Vorrichtung nach einem der vorhergehenden Ansprüche, in der der Druck in dem Plasma-Auslaßkreis (9) so eingestellt ist, daß er in dem Bereich von −2660 bis +2660 Pa (−20 mmHg bis +20 mmHg) liegt.

*Fig.1.*

Fig.2.

Fig.3.